# EUROPÄISCHE PATENTSCHRIFT

(11) **EP 1 499 253 B1**
(45) Veröffentlichungstag und Bekanntmachung des Hinweises auf die Patenterteilung: **04.06.2008**
(21) Anmeldenummer: 03747080.4
(22) Anmeldetag: 28.04.2003
(51) Int. Cl.: A61B 18/12

(54) **HOCHFREQUENZ-CHIRURGIEGENERATOR**
HIGH-FREQUENCY SURGERY GENERATOR
GENERATEUR CHIRURGICAL HAUTE FREQUENCE

(30) Priorität: 26.04.2002 DE 10218893
(43) Veröffentlichungstag der Anmeldung: 26.01.2005
(73) Patentinhaber: Storz Endoskop Produktions GmbH, 78532 Tuttlingen (DE)
(72) Erfinder: DANERS, Felix, CH-8200 Schaffhausen (CH); NOVAK, Pavel, CH-8234 Stetten (CH)
(74) Vertreter: Lohr, Georg
(86) Internationale Anmeldenummer: PCT/DE2003/001365
(87) Internationale Veröffentlichungsnummer: WO 2003/090634

(56) Entgegenhaltungen:
- WO-A-93/03677
- DE-A- 10 013 795
- US-A- 4 114 623

## Beschreibung

### Technisches Gebiet

Die Erfindung betrifft einen Generator zur Leistungserzeugung für die Hochfrequenzchirurgie. In der Hochfrequenzchirurgie wird menschliches oder tierisches Körpergewebe mittels elektrischem Strom geschnitten bzw. koaguliert. Die Hochfrequenzchirurgie ist insbesondere in Verbindung mit endoskopischen Operationstechniken äußerst vorteilhaft einsetzbar.

### Stand der Technik

Die Aufgabe der Hochfrequenzchirurgiegeneratoren ist es, die elektrische Energie für die Hochfrequenzchirurgie derart bereitzustellen, dass das gewünschte Operationsergebnis erreicht wird. Um Muskel- bzw. Nervenreizungen zu minimieren, liefern Hochfrequenzchirurgiegeneratoren hochfrequente Energie im Frequenzbereich über 300 kHz. Diese hochfrequente Energie wird meist mittels einer Elektrode in das Gewebe eingespeist. Am Punkt der Einspeisung tritt eine starke Erwärmung des die Elektrode umgebenden Gewebes auf. Wird in einem kurzen Zeitintervall eine hohe Energie zugeführt, so führt dies zu einem Verdampfen der Zellflüssigkeit und einem Aufplatzen der Zellen, so dass sich der Zellverband um die Elektrode auflöst. Die Elektrode kann sich nahezu frei durch das Gewebe bewegen. Wird über längere Zeit eine geringere Energie zugeführt, so führt dies zu einer Koagulation des Gewebes d. h. zu einer Gerinnung des Eiweißes. Die Zellen sterben hierbei ab und werden zu einer zähen Masse.

Grundsätzlich werden bezüglich der Einleitung der hochfrequenten Energie zwei Anordnungen unterschieden.

Bei der monopolaren Anordnung wird eine kleinflächige Schneide- bzw. Koagulationselektrode zur Stromeinleitung an Operationsort und eine großflächige "neutrale Elektrode" zur Stromausleitung an einem anderen Ort des Körpers des Patienten angeordnet. Die Elektrodenfläche ist hier so groß dimensioniert, dass es zu keiner nennenswerten Wärmeentwicklung an der Elektrode kommt.

Die bipolare Anordnung umfasst eine zweigeteilte Elektrode, bei der die Stromeinleitung sowie die Ausleitung am Operationsort erfolgt.

Der Dosierung der Energie kommt größte Bedeutung zu, da diese das Operationsergebnis unmittelbar beeinflusst. Gibt der Generator zu wenig Energie ab, so ist kein Schneiden möglich, wird zu viel Energie abgegeben, so werden die Schnittränder stark koaguliert, was wiederum zu einer erschwerten Abheilung bzw. erhöhtem Infektionsrisiko führt.

Somit ist es das Ziel, für einen reinen Schneidvorgang so wenig Energie die möglich und für einen kombinierten Schneide- bzw. Koagulationsvorgang die minimale, zur Koagulation benötigte Energie in das Gewebe einzubringen.

Um diese Energie zu minimieren, wird in der US-Patentschrift 4,114,623 ein Verfahren zur Regelung des Generatorstromes durch Beobachtungen des beim Schneiden auftretenden Lichtbogens offenbart.

Ein besonderes Problem stellt hier der Schnittbeginn bzw. der Übergang in eine andere Gewebeart mit anderen elektrischen Eigenschaften dar. Da beim Übergang in eine andere Gewebeart nahezu in die gleiche Aufgabenstellung wie beim Anschneiden vorliegt, wird nachfolgend nur noch auf Anschneiden Bezug genommen.

Wird mit zu hoher Leistung angeschnitten, so ergibt sich an der Anschnittstelle bereits eine unerwünschte Koagulation. Um diese Koagulation zu minimieren, wird in der DE 38 15 835 A1 eine Begrenzung der Generatorausgangsspannung vorgeschlagen. Diese verhindert ein Anschneiden mit zu hoher Generatorleistung. Wird stattdessen mit zu niedriger Leistung angeschnitten, so führt dieses zu keinem Schneidevorgang durch Eindringen der Elektrode in das Gewebe, sondern vielmehr zu einer unerwünschten Koagulation der Gewebeoberfläche. Diese erschwert auch ein weiteres Anschneiden. Um ein sicheres, gewebeunabhängiges Anschneiden zu gewährleisten, wird in der DE 41 35 184 A1 vorgeschlagen, zu Beginn des Anschneidens eine erhöhte Generatorleistung abzugeben. Diese erhöhte Leistungsabgabe kann dann bei Erkennung eines Lichtbogens auf den normal zum Schneiden benötigten Wert abgesenkt werden.

Die beiden hier vorgeschlagenen Maßnahmen zur Optimierung des Anschneidens schließen einander aus. So kann einerseits zur Vermeidung einer Koagulation durch zu hohe Anfangsleistung die Generatorleistung begrenzt werden, was aber, falls kein Anschneiden stattfindet, zu einer Koagulation führen kann. Andererseits kann um ein sicheres Anschneiden zu gewährleisten, mit erhöhter Leistung unter Inkaufnahme einer Koagulation angeschnitten werden.

Die Druckschrift DE-A-100 13 795 offenbart einen Hochfrequenzgenerator für die HF-Chirurgie umfassend:
- einen Leistungsgenerator zur Abgabe hochfrequenter Energie mit einer Grundfrequenz sowie einen Messabgriff zur Auskopplung einer Messgröße entsprechend der Ausgangsspannung, sowie einem Eingriff zur Vorgabe einer elektrischen Ausgangsgröße wie Ausgangspannung oder Ausgangsleistung;
- ein Filter zur Selektion von harmonischen Frequenzanteilen oberhalb der Grundfrequenz aus der Messgröße;
- Mittel zur Skalierung der harmonischen Frequenzanteile; und
- eine Signalisierung der skalierten Frequenzanteile mittels des Eingriffs an den Leistungsgenerator zur Steuerung des Leistungsgenerators derart, dass bei einer Erhöhung der Amplitude der skalierten harmonischen Frequenzanteile die Ausgangsspannung bzw. die Ausgangsleistung des Leistungsgenerators reduziert wird.

### Darstellung der Erfindung

Der Erfindung liegt die Aufgabe zugrunde, einen Hochfrequenzchirurgiegenerator bereitzustellen, welcher unabhängig von der Gewebeart ein sicheres Anschneiden ohne Koagulation der Anschnittstelle gewährleistet.

Eine erfindungsgemäße Lösung dieser Aufgabe ist im Patentanspruch 1 angegeben. Weiterbildungen der Erfindung sind Gegenstand der abhängigen Ansprüche.

Die erfindungsgemäße Vorrichtung umfasst einen Hochfrequenzgenerator (1) für die Hochfrequenzchirurgie. Dieser umfasst zumindest einen Leistungsgenerator (2), welcher zur Abgabe hochfrequenter Energie bei einer Grundfrequenz ausgelegt ist. Ein Messabgriff (3) dient zur Auskopplung einer Messgröße entsprechend der Ausgangsspannung oder dem Ausgangsstrom. Weiterhin ist ein Eingriff (4) zur Vorgabe einer elektrischen Ausgangsgröße wie Ausgangsspannung, Ausgangsstrom bzw. Ausgangsleistung vorgesehen. Grundsätzlich können diese Ausgangsgrößen auch auf die Ausgangsleistung bezogen werden. So ergibt sich bei einer bestimmten Lastimpedanz und gegebener Ausgangsspannung bzw. gegebenem Ausgangsstrom eine bestimmte Ausgangsleistung. Gerade die Höhe der in das Gewebe eingebrachten Leistung ist maßgeblich für die physikalische Auswirkung d.h. für Schneiden oder Koagulieren.
Erfindungsgemäß ist eine Normierungsschaltung (6) vorgesehen, welche einen Normierungsfaktor k derart ermittelt, dass die Messgröße multipliziert mit diesem Normierungsfaktor k einem vorgegebenen Sollwert entspricht. Anstelle der Messgröße kann bevorzugter Weise der Anteil der Grundfrequenz aus der Messgröße verwendet werden. Hierzu ist dann ein geeignetes Filter vorzusehen. Die Verwendung der Grundfrequenz ist vorteilhaft, wenn ein hoher Anteil an harmonischen Signalanteilen vorhanden ist, da diese sonst das Messergebnis verfälschen könnten. Weiterhin ist ein Filter (5) zur Selektion von harmonischen Frequenzanteilen oberhalb der Grundfrequenz aus der Messgröße vorgesehen. Diese harmonischen Frequenzanteile werden mittels eines Mittels (7) zur Skalierung mit dem zuvor ermittelten Normierungsfaktor k skaliert. Die derart skalierten harmonischen Frequenzanteile werden dann über den Eingriff (4) an den Leistungsgenerator (2) übermittelt. Es erfolgt eine Steuerung des Leistungsgenerators derart, dass eine Erhöhung der Amplitude der skalierten harmonischen Frequenzanteile eine verringerung der Ausgangsleistung oder auch Ausgangsspannung bzw. Ausgangsstrom des Leistungsgenerators bewirken.

Das Filter (5) kann auch aus mehreren Teilfiltern bestehen. Wesentlich ist hierbei die realisierte Übertragungsfunktion. So kann wahlweise eine einzelne harmonische, beispielsweise bei der 3-fachen oder auch 5-fachen Grundfrequenz selektiert werden. Ebenso können auch mehrere dieser Harmonischen oder auch breitere Frequenzbänder selektiert werden. Eine besonders einfache Ausgestaltung ergibt sich, wenn das Filter als Hochpassfilter, welches die Grundfrequenz unterdrückt und alle Frequenzanteile oberhalb der Grundfrequenz durchlässt, ausgebildet ist. Die optimale Anpassung des Filters erfolgte unter Berücksichtigung der spektralen Verteilung des Ausgangssignals des Generators. So kann beispielsweise im Falle eines unsymmetrischen Generatorausgangssignals, bei dem die ungeradzahligen Vielfachen der Grundfrequenz relativ hohe Amplituden aufweisen, eine Filtergestaltung sinnvoll sein, welche gerade diese unterdrückt. So ist grundsätzlich das Filter so zu dimensionieren, das ein Frequenzbereich selektiert wird, in welchem das Generatorausgangssignal - ohne dass ein Schneidvorgang stattfindet - relativ niedrige spektralen Anteile aufweist, in dem aber durch den beim Schneiden auftretenden Lichtbogen deutlich auswertbare Spektralanteile liegen. Diese liegen erfahrungsgemäß bei den ungeradzahligen Vielfachen der Grundfrequenz. Wesentlich bei der erfindungsgemäßen Ausgestaltung ist, dass durch die beschriebene Skalierung eine Unabhängigkeit von der Amplitude des Generatorsignals selbst erreicht wird. Somit kann ein einwandfreies Anschneidens bei vielfältigen Elektrodengeometrien unabhängig von der Querschnittfläche der Elektrode erreicht werden.

Um eine gute Regelungswirkung zu erreichen, ist es vorteilhaft, wenn der Hochfrequenzgenerator selbst bzw. falls dieser von einem Netzteil gespeist wird, dieses Netzteil eine hohe Stellgeschwindigkeit aufweist, so dass den Signalen, welche mittels des Eingriffs (4) übermittelt werden, schnell gefolgt werden kann. In Versuchen hat sich eine Zeitkonstante des Generators von kleiner 1 ms als vorteilhaft herausgestellt.

In einer besonders vorteilhaften Ausgestaltung der Erfindung weist die Normierungsschaltung (6) einen ersten Gleichrichter (13) zur Ermittlung des Spitzenwertes der Messgröße auf. Damit gibt der Normierungsfaktor k an, in welchen Wert der Spitzenwert der Messgröße skaliert werden muss, damit er den vorgegebenen Sollwert erreicht. Bei dieser Anordnung ist es besonders sinnvoll, vor der Normierung aus der Messgröße die Grundfrequenz zu selektieren, da dann kurzzeitig auftretende Störsignale, welche beispielsweise von hochfrequenten Harmonischen oder auch von Rauschen stammen, unterdrückt werden und nicht in die Spitzenwertmessung eingehen.

Eine weitere vorteilhafte Ausgestaltung der Erfindung weist in der Normierungsschaltung einen ersten Gleichrichter (8) zur Ermittlung des Effektivwertes beziehungsweise wahlweise des Mittelwerts der Messgröße auf. Damit gibt der Normierungsfaktor k an, in welchen Wert der Effektivwert der Messgröße skaliert werden muss, damit er den vorgegebenen Sollwert erreicht.

In einer weiteren vorteilhaft Ausgestaltung der Erfindung umfasst die Normierungsschaltung (6) einen Regelkreis. Dieser schwächt mittels eines ersten einstellbare Dämpfungsgliedes (9) die Messgröße derart ab, dass sie einem vorgegebenen Sollwert entspricht.

In diesen Ausführungen wird auf die Begriffe Dämpfungsglied, Abschwächer bzw. abschwächen Bezug genommen. Selbstverständlich umfassen diese Begriffe eine Skalierung im allgemeinen Sinne sowie auch die Verstärkung.

Eine weitere vorteilhafte Ausgestaltung der Erfindung besteht darin, dass das Filter (5) zur Selektion von harmonischen Frequenzanteilen hinter dem ersten einstellbaren Dämpfungsglied (9) angeordnet ist. Damit schwächt dieses Dämpfungsglied die gesamte Messgröße einschließlich der darin enthaltenen harmonischen Frequenzanteile ab. Es wird also zur Ermittlung des Skalierungsfaktors und zur Skalierung der harmonischen Frequenzanteile nur ein einziges Dämpfungsglied benötigt.

Eine weitere vorteilhafte Ausgestaltung der Erfindung besteht darin, dass zur Abschwächung der harmonischen Frequenzanteile ein zweites einstellbares Dämpfungsglied (10) vorgesehen ist, welches parallel mit dem ersten einstellbaren Dämpfungsglied (9) angesteuert wird. Somit entsprechen die Dämpfungen der Dämpfungsglieder einander, es werden also beide Signalpfade mit den gleichen Dämpfungen beaufschlagt. Durch die Aufteilung in zwei getrennte Signalpfade können diese getrennt dimensioniert und optimiert werden.

In einer weiteren vorteilhaften Ausgestaltung der Erfindung wird die Messgröße unmittelbar in ein Filter (5) zur Selektion von harmonischen Frequenzanteilen und in ein hierzu parallel geschaltetes Filter (11), welches die Grundfrequenz der Messgröße selektiert, eingespeist. Hinter den beiden Filtern sind die diesen zugeordneten Dämpfungsglieder angeordnet. Die Filter werden bevorzugt als passive Filter realisiert. Durch diese Ausgestaltung lässt sich eine extrem große Dynamik des Systems erreichen. Es befinden sich am Eingang der Schaltung die übersteuerungsfesten linearen, passiven Komponenten der Filter. Es werden nur die jeweils benötigten spektralen Anteile weitergeleitet, so dass den nachfolgenden aktiven Schaltungskomponenten wie Verstärker oder Dämpfungsglieder nur geringere Signalpegel eingespeist werden. Somit kann eine Übersteuerung dieser Komponenten, welche beispielsweise zu unerwünschten Harmonischen und damit zu einer Verfälschung des Messergebnisses führt, vermieden werden.

In einer weiteren vorteilhaften Ausgestaltung der Erfindung wird die Messgröße unmittelbar in das Filter (5) zur Selektion von harmonischen Frequenzanteilen mit einem nachgeschalteten zweiten Dämpfungsglied (10) eingespeist. Parallel zum Filter wird die Messgröße in ein erstes einstellbares Dämpfungsglied zur Abschwächung der Messgröße selbst eingespeist. Auch diese Ausgestaltung bietet eine verbesserte Dynamik, da dem zweiten Dämpfungsglied (10) ausschließlich die Signale mit niedrigem Pegel der harmonischen Frequenzanteile ohne die hohen Pegel der Grundfrequenz zugeführt werden.

In einer weiteren vorteilhaften Ausgestaltung der Erfindung ist eine Track & Hold - Schaltung vorgesehen. Diese Schaltung lässt während der Schneidens die aktuellen Signalwerte ungehindert passieren. Wird diese Schaltung ein Ende des Schnittes signalisiert, so speichert sie den letzten Signalwert solange, bis das Schneiden wieder aufgenommen wird. Eine solche Zwischenspeicherung der aktuellen Werte ist besonders sinnvoll bei Zeitverläufen mit kurzzeitigen Unterbrechungen wie dem Koagulierenden Schneiden, bei dem jeweils kurze Phasen mit Schnitten und Koagulationen abwechselnd auftreten. Eine solche Track & Hold - Schaltung wird bevorzugt am Eingang der Anordnung bzw. unmittelbar hinter dem Messabgriff des Generators angebracht. Insbesondere bei Regelverstärkern mit integralem Verhalten sind auch diese bevorzugte Weise mit einer Track & Hold - Schaltung auszurüsten. Je nach Auslegung der Schaltung kann eine oder mehrere Track & Hold - Schaltungen an verschiedenen Orten angebracht werden.

### Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben.

Fig. 1 zeigt in allgemeiner Form schematisch eine erfindungsgemäße Vorrichtung.

Fig. 2 zeigt beispielhaft eine Ausführungsform mit einer besonders hohen Dynamik.

Fig. 3 zeigt beispielhaft eine besonders einfache Ausgestaltung der Erfindung.

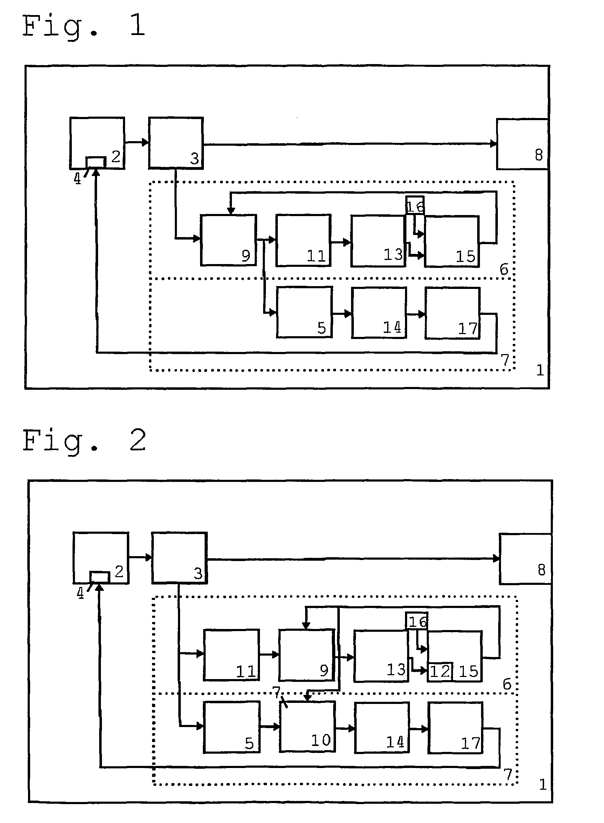

In der Fig. 1 ist eine erfindungsgemäße Vorrichtung beispielhaft abgebildet. Ein Generator für die Hochfrequenzchirurgie (1) (Hochfrequenzchirurgiegenerator) umfasst einen Leistungsgenerator (2), welcher die hochfrequente Leistung an die Ausgangsbuchsen (8) abgibt. In Verbindung mit dem Ausgangskreis befindet sich ein Messabgriff (3), welcher zur Auskopplung einer Messgröße entsprechend der Ausgangsspannung oder dem Ausgangsstrom des Leistungsgenerators dient. Dieser Messabgriff kann in den Leistungsgenerator selbst integriert sein und beispielsweise die Spannung am Ausgangstransformator abgreifen. Vorteilhafter ist jedoch eine Anordnung möglichst nahe an den Ausgangsbuchsen, um die Signale möglichst unbeeinflusst von internen Störungen bzw. parasitären Verkopplungen zu erfassen. Welche Ausgangsgröße gemessen wird, hängt von der Gestaltung der Ausgangsbeschaltung des Leistungsgenerators und der an die Ausgangsbuchsen angeschlossenen Last ab. Eine Messung des Stroms hat sich jedoch als besonders vorteilhaft herausgestellt, da hier im Falle von Lichtbogen in den meisten Fällen die Signalamplituden der Harmonischen am größten sind.

Die Messgröße wird vom Messabgriff (3) der Normierungsschaltung (6) zugeführt, welche eine entsprechende Normierung harmonischer Frequenzanteile der Grundfrequenz durchführt. Diese Signale werden dann über die Mittel zur Skalierung der harmonischen Frequenzanteile und über einen Eingriff (4) dem Leistungsgenerator (2) zur Steuerung einer Ausgangsgröße zugeführt. Eine steuerbare Ausgangsgröße ist beispielsweise die Leistung, der Strom und auch die Spannung des Leistungsgenerators. Ein besonders stabiles Verhalten ergibt sich im Falle der Steuerung der Spannung des Leistungsgenerators, da bei konstantem Gewebe die zum Schneiden benötigte Spannung unabhängig von Elektrodengeometrie, Schnitttiefe und Schnittgeschwindigkeit ist. Somit muss nur im Falle von Änderungen der Gewebebeschaffenheit ein neuer Spannungswert eingestellt werden. Damit ist in diesem Falle die Regelaktivität am geringsten. Es lässt sich so die stabilste Regelung realisieren.

In diesem Beispiel werden nun die Signale vom Messabgriff (3) mittels eines ersten einstellbaren Dämpfungsgliedes (9) an ein Filter (11) zur Selektion der Grundfrequenz der Messgröße weitergeleitet. Die derart gefilterten Signale werden über einen ersten Gleichrichter (13) einem Regelverstärker (15) zugeführt. Dieser vergleicht die Signale mit einem vorgegebenen Sollwert (16) und stellt entsprechende Steuersignale für das erste einstellbare Dämpfungsglied zur Einstellung der Dämpfung zur Verfügung. Durch den Regler wird die Größe (Amplitude, Effektivwert, etc.) des Signals auf der Grundfrequenz auf einen konstantem Wert, entsprechend dem Sollwert geregelt.

Ein Filter (5) zur Selektion von harmonischen Frequenzanteilen wird auch von den Ausgangsignalen des ersten Dämpfungsgliedes (9) gespeist. Somit werden die harmonischen Frequenzanteile ebenso wie die Grundfrequenz vom ersten Dämpfungsglied (9) abgeschwächt und somit auch mit dem gleichen Normierungsfaktor k normiert. Die Ausgangssignale des Filters (5) werden in einem zweiten Gleichrichter (14) zur Gleichrichtung und einem nachfolgenden Verstärker (17) weiterverarbeitet und anschließend mittels des Eingriffs (4) dem Leistungsgenerator (1) zugeführt. In vielen Fällen kann auch der Verstärker (17) weggelassen werden. Er dient hier lediglich zur Impedanzanpassung bzw. Skalierung mit einem konstantem Faktor, um das Ausgangssignal des Gleichrichters an den Leistungsgenerator anzupassen.

In Fig. 2 ist eine Ausführungsform der Erfindung mit besonders hoher Dynamik dargestellt.
Hierbei wird das Ausgangssignal des Messabgriffs (3) direkt dem Filter (11) zur Selektion der Grundfrequenz der Messgröße sowie dem in hierzu parallel geschalteten Filter (5) zur Selektion von harmonischen Frequenzanteilen zugeführt. Es ergeben sich somit zwei getrennte Signalpfade für die Grundfrequenz und für die harmonischen. Diese können nun getrennt entsprechend den auftretenden Signalamplituden behandelt werden. So besteht eine wesentlich geringere Gefahr der Erzeugung von Harmonischen durch Übersteuerung in Schaltungskomponenten. Im ersten Signalpfad wird die vom Filter (11) ausgefilterte Grundfrequenz weiter über ein erstes einstellbares Dämpfungsglied (9) dem Regelverstärker (15) zugeführt. Dieser steuert nun parallel das erste einstellbare Dämpfungsglied (9) sowie das zweite einstellbare Dämpfungsglied (10) an. Somit weisen beide Dämpfungsglieder den gleichen oder zumindest proportionale Dämpfungsfaktoren auf. Die derart durch das zweite einstellbare Dämpfungsglied (10) gedämpften harmonischen Anteile werden weiter über einen zweiten Gleichrichter (14) und einem optionalen Verstärker (17) mittels des Eingriffs (4) dem Leistungsgenerator (2) zur Steuerung zugeführt. Um eine einwandfreie Regelung auch bei gepulsten bzw. unterbrochenen Schneidvorgängen sicherzustellen, ist eine Track & Hold Schaltung (12) vorgesehen. Diese wird vorzugsweise am Eingang des Regelverstärkers (15) angeordnet.

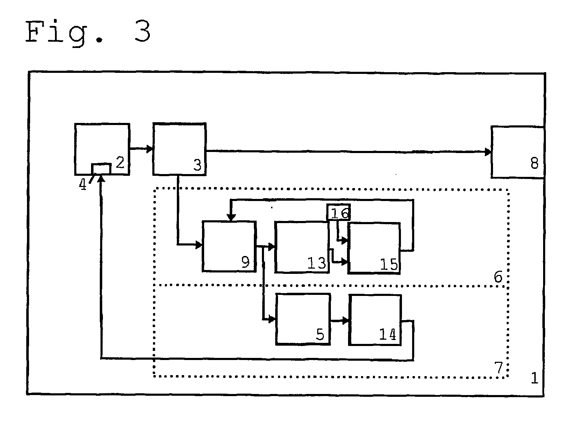

In Fig. 3 ist eine weitere Ausführungsform der Erfindung, welche mit besonders wenig Aufwand realisiert werden kann, dargestellt. Hierin wurden gegenüber Fig. 1 das Filter (11) zur Selektion der Grundfrequenz der Messgröße sowie der Verstärker (17) weggelassen. Enthält die Messgröße nur geringe harmonisch Anteile zusammen mit den Signalen der Grundfrequenz, so kann das Filter (11) ohne wesentliche Beeinträchtigung weggelassen werden.

### Bezugszeichenliste

- 1: Hochfrequenzgenerator
- 2: Leistungsgenerator
- 3: Messabgriff
- 4: Eingriff zur Vorgabe einer elektrischen Ausgangsgröße
- 5: Filter zur Selektion von harmonischen Frequenzanteilen
- 6: Normierungsschaltung
- 7: Mittel zur Skalierung der harmonischen Frequenzanteile
- 8: Ausgangsbuchsen
- 9: erstes einstellbares Dämpfungsglied
- 10: zweites einstellbares Dämpfungsglied
- 11: Filter zur Selektion der Grundfrequenz der Messgröße
- 12: Track & Hold - Schaltung
- 13: Erster Gleichrichter
- 14: Zweiter Gleichrichter
- 15: Regelverstärker
- 16: Sollwert
- 17: Verstärker

## Patentansprüche

1. Hochfrequenzgenerator (1) für die Hochfrequenzchirurgie umfassend einen Leistungsgenerator (2) zur Abgabe hochfrequenter Energie mit einer Grundfrequenz sowie einen Messabgriff (3) zur Auskopplung einer Messgröße entsprechend der Ausgangsspannung oder dem Ausgangsstrom, sowie mindestens einem Eingriff (4) zur Vorgabe einer elektrischen Ausgangsgröße wie Ausgangsspannung, Ausgangsstrom bzw. Ausgangsleistung,
wobei
- ein Filter (5) zur Selektion von harmonischen Frequenzanteilen oberhalb der Grundfrequenz aus der Messgröße und
- eine Normierungsschaltung (6) vorgesehen ist, welche einen Normierungsfaktor k derart ermittelt, dass die Messgröße oder der Anteil der Grundfrequenz aus der Messgröße multipliziert mit dem Normierungsfaktor k einem vorgegebenen Sollwert entspricht und weiterhin
- Mittel (7) zur Skalierung der harmonischen Frequenzanteile mit dem Normierungsfaktor k vorgesehen sind und
- eine Signalisierung der skalierten harmonischen Frequenzanteile mittels des Eingriffs (4) an den Leistungsgenerator (2) zur Steuerung des Leistungsgenerators derart vorgesehen ist, dass bei einer Erhöhung der Amplitude der skalierten harmonischen Frequenzanteile die Ausgangsspannung, der Ausgangstrom oder die Ausgangsleistung des Leistungsgenerators reduziert wird.

2. Vorrichtung nach Anspruch 1,
**dadurch gekennzeichnet, dass** die Normierungsschaltung (6) einen ersten Gleichrichter (13) zur Ermittlung des Spitzenwertes der Messgröße aufweist und den Normierungsfaktor k bezüglich des Spitzenwertes der Messgröße ermittelt.

3. Vorrichtung nach Anspruch 1,
**dadurch gekennzeichnet, dass** die Normierungsschaltung (6) einen ersten Gleichrichter (13) zur Ermittlung des Effektivwertes bzw. Mittelwertes der Messgröße aufweist und den Normierungsfaktor k bezüglich des Effektivwertes der Messgröße ermittelt.

4. Vorrichtung nach einem der Ansprüche 1 bis 3,
**dadurch gekennzeichnet, dass** die Normierungsschaltung (6) einen Regelkreis umfasst, welcher mittels eines ersten einstellbaren Dämpfungsgliedes (9) die Messgröße derart abschwächt, dass sie einem vorgegebenen Sollwert entspricht.

5. Vorrichtung nach Anspruch 4,
**dadurch gekennzeichnet, dass** das Filter (5) zur Selektion von harmonischen Frequenzanteilen hinter dem einstellbaren Dämpfungsglied (9) angeordnet ist, so dass mit dem einstellbaren Dämpfungsglied (9) die gesamte Messgröße einschließlich der harmonischen Frequenzanteile abgeschwächt wird.

6. Vorrichtung nach Anspruch 4,
**dadurch gekennzeichnet, dass** zur Abschwächung der harmonischen Frequenzanteile ein zweites einstellbares Dämpfungsglied (10) vorgesehen ist, welches parallel mit dem ersten einstellbaren Dämpfungsglied (9) angesteuert wird, so dass die Dämpfungen der Dämpfungsglieder einander entsprechen.

7. Vorrichtung nach Anspruch 6,
**dadurch gekennzeichnet, dass** die Messgröße unmittelbar in das Filter (5) zur Selektion von harmonischen Frequenzanteilen und in einen hierzu parallel geschaltetes Filter (11), welches die Grundfrequenz der Messgröße selektiert, gespeist wird und dass hinter diesen Filtern die entsprechend zugeordneten einstellbaren Dämpfungsglieder (9, 10) angeordnet sind.

8. Vorrichtung nach Anspruch 6,
**dadurch gekennzeichnet, dass** die Messgröße unmittelbar in das Filter (5) zur Selektion von harmonischen Frequenzanteilen mit nachgeschaltetem zweiten Dämpfungsglied (10) und in ein hierzu parallel geschaltetes erstes Dämpfungsglied (9), welches die Messgröße selbst abschwächt, gespeist wird.

9. Vorrichtung nach einem der vorhergehenden Ansprüche,
**dadurch gekennzeichnet, dass** eine Track & Hold - Schaltung (12) vorgesehen ist, welche während des Schneidens die aktuellen Signalwerte durchlässt und bei kurzzeitigen Unterbrechungen des Schneidens den letzten Signalwert solange speichert, bis das Schneiden wieder fortgesetzt wird.

## Claims

1. A high-frequency generator (1) for high-frequency surgery, comprising a power generator (2) for emitting high-frequency energy with a fundamental frequency and a measurement tap (3) for tapping a measured quantity according to the output voltage or output current, and at least one override (4) for predetermining an electric output quantity such as output voltage, output current or output power, with
- a filter (5) being provided for selecting harmonic frequency components above the fundamental frequency from the measured quantity, and
- normalization circuit (6) which determines a normalization factor k in such a way that the measured quantity or the fundamental frequency component of measured quantity multiplied with normalization factor k corresponds to a predetermined setpoint value, and further
- means (7) being provided for scaling the harmonic frequency components with the normalization factor k, and
- a signaling of the scaled harmonic frequency components by means of the override (4) to the power generator (2) for controlling the power generator being provided in such a way that in the case of an increase of the amplitude of the scaled harmonic frequency components the output voltage, output current or output power of the power generator is reduced.

2. An apparatus according to claim 1, **characterized in that** the normalization circuit (6) has a first rectifier (13) for determining the peak value of the measured quantity and determines the normalization factor k concerning the peak value of the measured quantity.

3. An apparatus according to claim 1, **characterized in that** the normalization circuit (6) comprises a first rectifier (13) for determining the effective value or mean value of the measured quantity and determines the normalization factor k concerning the effective value of the measured quantity.

4. An apparatus according to one of the claims 1 to 3, **characterized in that** the normalization circuit (6) comprises a closed-loop control circuit which by means of a first adjustable attenuator (9) attenuates the measured quantity in such a way that it corresponds to a predetermined setpoint value.

5. An apparatus according to claim 4, **characterized in that** the filter (5) for selecting harmonic frequency components is arranged behind the adjustable attenuator (9), so that the entire measured quantity including the harmonic frequency components is attenuated with the adjustable attenuator (9).

6. An apparatus according to claim 4, **characterized in that** for attenuating the harmonic frequency components a second adjustable attenuator (10) is provided which is triggered in parallel with the first adjustable attenuator (9) in such a way that the attenuations of the attenuators correspond to one another.

7. An apparatus according to claim 6, **characterized in that** the measured quantity is supplied directly to the filter (5) for selecting harmonic frequency components and to a filter (11) which is connected parallel thereto and which selects the fundamental frequency of the measured quantity, and that the respectively associated adjustable attenuators (9, 10) are arranged behind said filters.

8. An apparatus according to claim 6, **characterized in that** the measured quantity is supplied directly to the filter (5) for selecting harmonic frequency components with a second attenuator (10) connected in outgoing circuit and to a first attenuator (9) which is connected in parallel thereto and which attenuates the measured quantity itself.

9. An apparatus according to one of the preceding claims, **characterized in that** a track & hold circuit (12) is provided which during the cutting allows the current signal values to pass and during brief interruptions of the cutting stores the last signal value for such a time until the cutting is continued again.

## Revendications

1. Générateur de hautes fréquences (1) pour la chirurgie à haute fréquence, comprenant un générateur de puissance (2) destiné à fournir une énergie à haute fréquence à une fréquence de base ainsi qu'une prise de mesure (3) pour la dérivation d'une grandeur de mesure correspondant à la tension de sortie ou à l'intensité de sortie, ainsi qu'au moins une entrée (4) pour l'indication d'une grandeur de sortie électrique telle que la tension de sortie, l'intensité de sortie ou la puissance de sortie,
dans lequel sont prévus
- un filtre (5) pour la sélection de fractions de fréquence harmoniques au-dessus de la fréquence de base dans la grandeur de mesure et
- un circuit de normalisation (6) qui détermine un facteur de normalisation k de telle manière que la grandeur de mesure ou la fraction de la fréquence de base issue de la grandeur de mesure multipliée par le facteur de normalisation k corresponde à une valeur de consigne prédéterminée, et
- il est prévu en outre des moyens (7) pour la mise à l'échelle des fractions de fréquence harmoniques avec le facteur de normalisation k et
- une signalisation des fractions de fréquence harmoniques mises à l'échelle par l'entrée (4) au générateur de puissance (2) est prévue pour la commande du générateur de puissance, de telle manière que si l'amplitude des fractions de fréquence harmoniques mises à l'échelle augmente, la tension de sortie, l'intensité de sortie ou la puissance de sortie du générateur de puissance est réduite.

2. Dispositif selon la revendication 1, **caractérisé en ce que** le circuit de normalisation (6) possède un premier redresseur (13) pour déterminer la valeur de crête de la grandeur de mesure et détermine le facteur de normalisation k par rapport à la valeur de crête de la grandeur de mesure.

3. Dispositif selon la revendication 1, **caractérisé en ce que** le circuit de normalisation (6) présente un premier redresseur (13) pour déterminer la valeur effective ou la valeur moyenne de la grandeur de mesure et déterminer le facteur de normalisation k par rapport à la valeur effective de la grandeur de mesure.

4. Dispositif selon la revendication 1 à 3, **caractérisé en ce que** le circuit de normalisation (6) comprend un circuit de régulation qui affaiblit la grandeur de mesure au moyen d'un premier élément atténuateur réglable (9), de telle sorte qu'elle corresponde à une grandeur de mesure prédéterminée.

5. Dispositif selon la revendication 4, **caractérisé en ce que** le filtre (5) pour la détection de fractions de fréquence harmoniques est disposé en aval de l'élément atténuateur réglable (9), de telle sorte que l'élément atténuateur réglable (9) atténue l'ensemble de la grandeur de mesure, y compris les fractions de fréquence harmoniques.

6. Dispositif selon la revendication 4, **caractérisé en ce que** pour atténuer les fractions de fréquence harmoniques, il est prévu un deuxième élément atténuateur réglable (10), qui est activé en parallèle avec le premier élément atténuateur réglable (9), de telle manière que les atténuations des éléments atténuateurs se correspondent.

7. Dispositif selon la revendication 6, **caractérisé en ce que** la grandeur de mesure est entrée directement dans le filtre (5) pour la sélection des fractions de fréquence harmoniques et dans un filtre (11) monté en parallèle avec celui-ci, qui sélectionne les fréquence de base de la grandeur de mesure, et **en ce que** les éléments atténuateurs réglables (9, 10) affectés de manière correspondante sont disposés en aval de ces filtres.

8. Dispositif selon la revendication 6, **caractérisé en ce que** la grandeur de mesure est entrée directement dans le filtre (5) pour la sélection des fractions de fréquence harmoniques en aval duquel le deuxième élément atténuateur (10) est monté et dans un premier élément atténuateur (9) monté en parallèle avec celui-ci, qui atténue la grandeur de mesure elle-même.

9. Dispositif selon l'une des revendications précédentes, **caractérisé en ce qu'**il est prévu un circuit de poursuite et de maintien (12) qui laisse passer les valeurs de signal actuelles pendant l'incision et qui enregistre la dernière valeur de signal, en cas d'interruption de courte durée de l'incision, jusqu'à ce que l'incision soit poursuivie.
